# EUROPEAN PATENT APPLICATION

(11) **EP 3 912 647 A1**
(43) Date of publication of application: **24.11.2021**
(21) Application number: 21175461.9
(22) Date of filing: 21.05.2021
(51) Int. Cl.: A61L 2/10, A61L 2/24, A61L 9/20, B64D 11/00

(54) **ANTIVIRAL SYSTEMS AND METHOD**

(30) Priority: 21.05.2020 US 202063028498 P
(71) Applicant: Goodrich Corporation, Charlotte, NC 28217-4578 (US)
(72) Inventor: GURVICH, Mark R., Middletown, CT 06457 (US)
(74) Representative: Dehns

(57) **Abstract**

An antiviral system comprises, an interior space (102), and an ultra-violet (UV) illuminator system (106) fixedly mounted within the interior space for illumination of surfaces (104) within the interior space for destruction of pathogens on the surfaces. A plurality of seats (122) can be arranged within the interior space such that the UV illuminator system is configured to illuminate full or partial surfaces of individual seats of the plurality of seats.

## Description

### CROSS-REFERENCE TO RELAED APPLICATIONS

This application claims priority to and the benefit of U.S. Provisional Patent Application No. 63/028,498 filed May 21, 2020.

### BACKGROUND

### 1. Field

This disclosure relates generally to antiviral systems and methods, and more particularly to antiviral systems and methods for interior spaces such as aircraft interiors.

### 2. Description of Related Art

Large interior spaces are among some of the most likely places to harbor the spread of pathogens due to close proximity of people and perhaps coming from different world-wide locations. Pathogens can persist on surfaces and existing sterilization methods of cleaning such interior spaces are primarily manual (e.g. spray and wipe), can be labor intensive, expensive, time-consuming, and may have a low probability of full sterilization.

The conventional techniques have been considered satisfactory for their intended purpose. However, there is an ever present need for improved systems and methods for more time/cost efficient and more reliable sterilization of large enclosed spaces. This disclosure provides a solution for this need.

### SUMMARY

An antiviral system comprises, an interior space, and an ultra-violet (UV) illuminator system fixedly mounted within the interior space for illumination of surfaces within the interior space for destruction or reduction of effect of pathogens on the surfaces. A plurality of seats can be arranged within the interior space, wherein the UV illuminator system is configured to illuminate full or partial surfaces of individual seats of the plurality of seats.

In certain embodiments, the interior space can form an interior of a passenger vehicle selected from the group consisting of: an aircraft, a ship, a bus, a trolleybus, a tram, a car, a train, a funicular or cable car and an elevator car. In certain embodiments, the interior space can form an interior of a stationary building selected from the group consisting of: wherein the interior space forms an interior of a stationary building selected from the group consisting of: a theater or a movie theater, an auditorium or a class room, a stadium or a sport-training room, a concert or a circus hall, and a restaurant including service and/or preparation areas. The illuminator system can include a plurality of UV emitting lamps which can be configured to emit at least one of UVA, UVB, and/or UVC.

An aircraft antiviral system can include an aircraft defining an aircraft interior, and an ultra-violet (UV) illuminator system fixedly mounted within the aircraft interior for illumination of surfaces within the interior space for destruction or reduction of effect of pathogens on the surfaces. The aircraft interior can include at least one of a cockpit, passenger cabin, lavatory, and/or galley.

A plurality of seats arranged within the aircraft interior, wherein the UV illuminator system is configured to illuminate full or partial surfaces of individual seats of the plurality of seats. The illuminator system can include a plurality of UV emitting lamps configured to emit at least one of UVA, UVB, and/or UVC.

In certain embodiments, the plurality of UV emitting lamps can be disposed along a length of an overhead bin array. In certain embodiments, the plurality of UV emitting lamps can be disposed along a footpath of an aisle. In certain embodiments, the plurality of UV emitting lamps can be disposed along a side panel of the aircraft interior space bounding an outer perimeter of a passenger row. In certain embodiments, the plurality of UV emitting lamps can be disposed along a ceiling of the aircraft interior. In embodiments, the plurality of UV emitting lamps are positioned such that UV emitted from the plurality of UV emitting lamps is incident upon high touchpoint surfaces of the interior of the aircraft.

The illuminator system can further include a controller operatively connected to the illuminator system. The controller includes machine readable instructions configured to cause the controller to perform a method. The method can include any one or all of: activating the illuminator system, sterilizing the aircraft interior with the illuminator system, deactivating the illuminator system after a predetermined duration of time, boarding the aircraft, completing aircraft flight and/or service, de-boarding the aircraft, and repeating the method.

A method for sterilizing an interior of an aircraft can include activating a ultra-violet (UV) illuminator system, the UV illuminator system fixedly mounted within the interior of the aircraft for illumination of surfaces within the interior aircraft for destruction or reduction of effect of pathogens on the surfaces of the interior. The method can include boarding the aircraft with a plurality of passengers and/or crew, completing a flight and/or service of the aircraft, and de-boarding the aircraft. The method can further include activating the UV illuminator system and repeating the method.

These and other features of the systems and methods of the subject disclosure will become more readily apparent to those skilled in the art from the following detailed description taken in conjunction with the drawings.

### BRIEF DESCRIPTION OF THE DRAWINGS

So that those skilled in the art to which the subject disclosure appertains will readily understand how to make and use the devices and methods of the subject disclosure without undue experimentation, other embodiments thereof will be described in detail herein below with reference to certain figures, wherein:
Fig. 1 is a schematic perspective view of an embodiment of a an interior space constructed in accordance with the present disclosure, showing an antiviral system;
Fig. 2 is a schematic plan view of the interior space of a representative aircraft;
Fig. 3 is a schematic axial cross-sectional view of the interior space, showing illuminators;
Fig. 4 is a schematic perspective view of the interior space, showing the illuminators; and
Fig. 5 is a diagram, schematically showing an embodiment of a method in accordance with the present disclosure.

### DETAILED DESCRIPTION

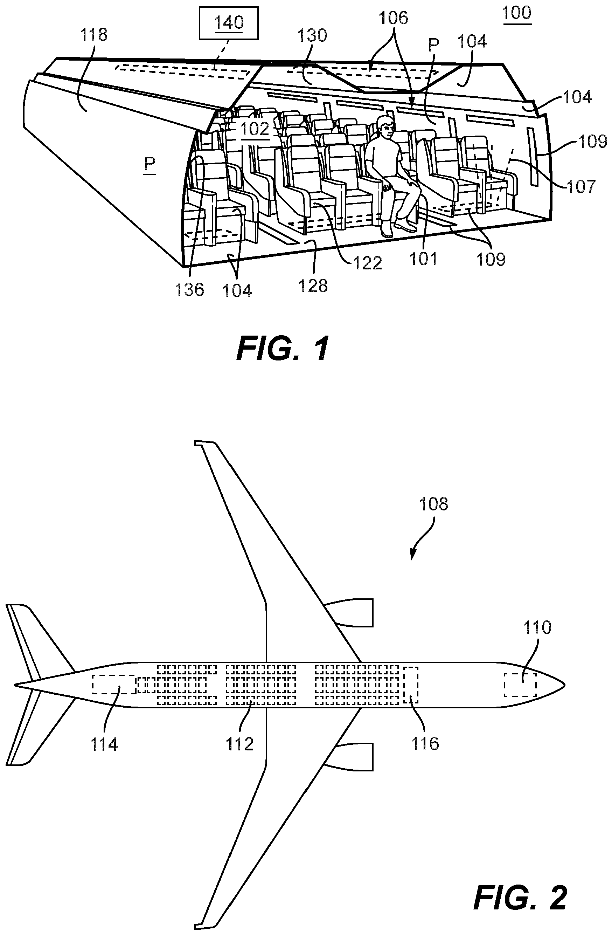

Reference will now be made to the drawings wherein like reference numerals identify similar structural features or aspects of the subject disclosure. For purposes of explanation and illustration, and not limitation, a partial view of an embodiment of a system in accordance with the disclosure is shown in Fig. 1 and is designated generally by reference character 100. Other embodiments of systems in accordance with the disclosure, or aspects thereof, are provided in Figs. 2-5, as will be described. The systems and methods described herein can be used to improve sterilization of interior spaces.

In certain embodiments, such as shown in Fig. 1, an antiviral system 100 can include an interior space 102 having a plurality of surfaces 104, and an ultra-violet (UV) illuminator system 106 fixedly mounted in any suitable location within the interior space 102. The UV illuminator system 106 can be configured to illuminate with UV 107 the surfaces 104 for destruction of pathogens on the surfaces or reduction of their pathogenic effects. It is contemplated that the UV illuminator 106 system can include, for example, black light fluorescent tubes, short-wave ultra-violet lamps 109 (e.g. in a UV LED strip), incandescent lamps, or the like. The UV illuminator system 106 can emit any one or all spectrums of UV radiation including UVA, UVB, and/or UVC. UVA is abbreviation of ultraviolet A and usually covers wavelength within 315-400 nm. UVA is also often known as long-wave UV UVB is abbreviation of ultraviolet B and usually covers wavelength within 280-315 nm and is also often known as medium-wave UV. UVC is abbreviation of ultraviolet C and usually covers wavelength within 100-280 nm and is also often known as short-wave UV

It is contemplated the antiviral system 100 can be used in a plurality of applications, for example, the interior space 102 can form an interior of a passenger vehicle selected from the group consisting of: an aircraft 1, a ship, a bus, a train, a trolleybus, a tram, a car, a train, a funicular or cable car and an elevator car. If the interior space is an interior of an aircraft 108 (e.g., as shown in Fig. 2), the aircraft interior can include at least one of a cockpit 110, passenger unit 112, a lavatory 114, and/or galley 116, among others. Variants of aircrafts can also include fixed-wing aircrafts and rotary-wing (helicopter) aircrafts. In certain embodiments, the interior space can form an interior of a stationary building 118 (e.g. as shown in Fig. 1) selected from the group consisting of: a theater or a movie theater, an auditorium or a class room, a stadium or a sport-training room, a concert or a circus hall, and a restaurant, including service and/or preparation areas, among others.

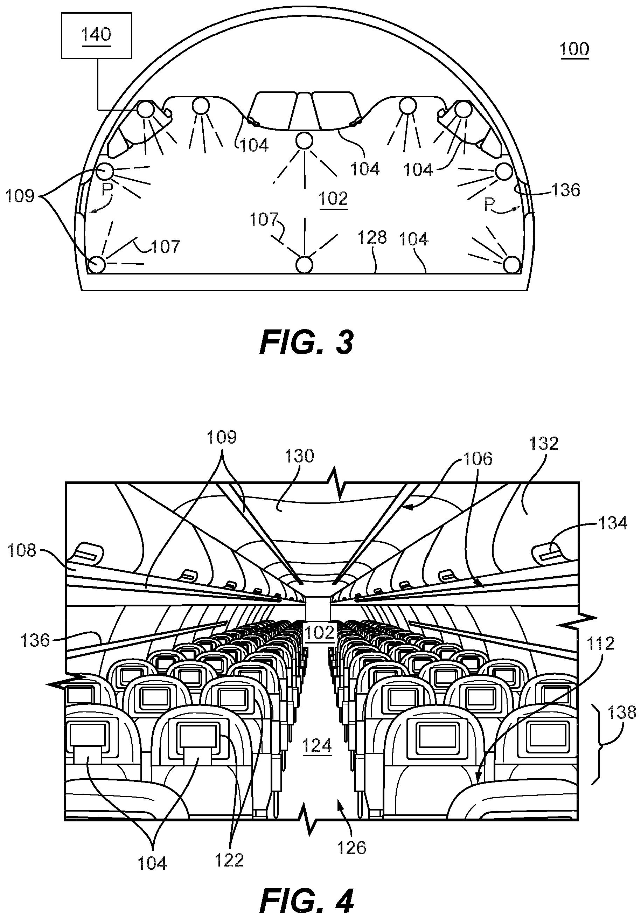

In embodiments, for example shown in Fig. 3, the plurality of UV emitting lamps 109 can positioned such that UV illumination 107 emitted from the plurality of UV emitting lamps 109 is incident upon high touchpoint surfaces (e.g., surfaces touched on almost every use of the interior space, as opposed to other surfaces which are rarely if ever touched). For example, in certain embodiments, a plurality of seats 122 can be arranged within the interior space 102, for holding users 101 of the interior space 102, such that the UV illuminator system 106 is configured to illuminate a full or partial surfaces 104 of individual seats 122. In certain embodiments, the plurality of UV emitting lamps 109 can be disposed along a footpath 124 of an aisle 126, for example under the seats 122, illuminating a floor 128 of the interior space 102 within the seat unit 112 and the aisle 126. It is also contemplated that the UV lamps disposed in the foot path 124 can illuminate upwards, filling the seat unit 112 and aisle 126. In certain embodiments, the plurality of UV emitting lamps 109 can be disposed along and/or throughout a ceiling 130 of the interior space 102.

In certain embodiments, such as shown in Fig. 4, for example if the interior space 102 is an aircraft 1 or passenger vehicle interior, the plurality of UV emitting lamps 109 can be disposed along a length of an overhead bin array 132, illuminating both the overhead bin handles 134, as well as illuminating a passenger unit 112 below the UV lamps 109. In certain embodiments, the plurality of UV emitting lamps 109 can be disposed along a side panel 136 of the aircraft interior space 102, for example bounding an outer perimeter P (Fig. 1) of a passenger row 138, where passengers 101 are likely to lean as well as adjust window shades, for example.

The illuminator system 106 can further include a controller 140 operatively connected to the UV illuminator system 106. The controller 140 can connected to the UV illuminator system 106 in any suitable manner, for example via hard wired, or wireless connection. The controller 140 can be located inside the interior space 102 or in a remote location from the UV illuminator system 106, for example in a control room, a cockpit, a galley, or outside the interior space 102. The illuminator system can be implemented within a given vehicle as a single fully connected system or as a combination of several independent illuminator sub-systems.

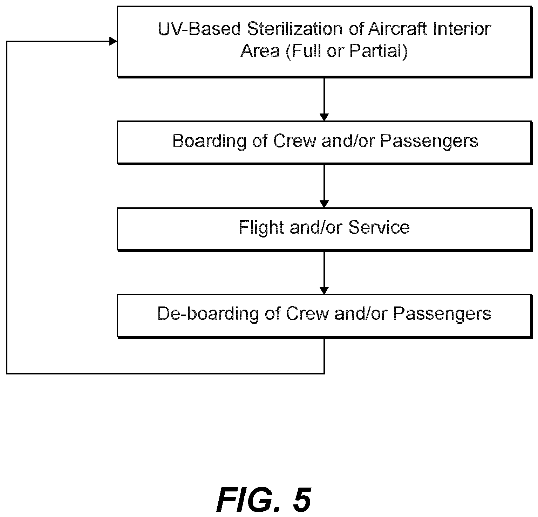

The controller 140 includes machine readable instructions configured to cause the controller 140 to perform a method, for example as shown in Fig. 5. The method can include any one or all of: activating the UV illuminator system 106, sterilizing the interior space 102 with the UV illuminator system 106, deactivating the UV illuminator system 106 after a predetermined duration of time (e.g. any suitable duration of time to disinfect surfaces 104 or longer), boarding or filling the interior space 102 with users 101, e.g., crew or/and passengers, completing an activity or service in or using the interior space 102, de-boarding or emptying the interior space 102 of users 101, and repeating the method as necessary for any number of additional activities and/or services.

Shown in Fig. 5, a method for sterilizing the interior 102 of an aircraft 108 can include activating the ultra-violet (UV) illuminator system 106, boarding the aircraft 108 with a plurality of passengers and/or crew 101, completing a flight and/or service of the aircraft 108, de-boarding the aircraft 108, activating the UV illuminator system 106 and repeating the method for additional flights and/or services.

As will be appreciated by those skilled in the art, aspects of the present disclosure may be embodied as a system, method or computer program product. Accordingly, aspects of this disclosure may take the form of an entirely hardware embodiment, an entirely software embodiment (including firmware, resident software, micro-code, etc.), or an embodiment combining software and hardware aspects, all possibilities of which can be referred to herein as a "circuit," "module," or "system." A "circuit," "module," or "system" can include one or more portions of one or more separate physical hardware and/or software components that can together perform the disclosed function of the "circuit," "module," or "system", or a "circuit," "module," or "system" can be a single self-contained unit (e.g., of hardware and/or software). Furthermore, aspects of this disclosure may take the form of a computer program product embodied in one or more computer readable medium(s) having computer readable program code embodied thereon.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electromagnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium may be transmitted using any appropriate medium, including but not limited to wireless, wireline, optical fiber cable, RF, etc., or any suitable combination of the foregoing.

Computer program code for carrying out operations for aspects of this disclosure may be written in any combination of one or more programming languages, including an object oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of this disclosure may be described above with reference to flowchart illustrations and/or block diagrams of methods, apparatus (systems) and computer program products according to embodiments of this disclosure. It will be understood that each block of any flowchart illustrations and/or block diagrams, and combinations of blocks in any flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in any flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified herein.

Those having ordinary skill in the art understand that any numerical values disclosed herein can be exact values or can be values within a range. Further, any terms of approximation (e.g., "about", "approximately", "around") used in this disclosure can mean the stated value within a range. For example, in certain embodiments, the range can be within (plus or minus) 20%, or within 10%, or within 5%, or within 2%, or within any other suitable percentage or number as appreciated by those having ordinary skill in the art (e.g., for known tolerance limits or error ranges).

The articles "a", "an", and "the" as used herein and in the appended claims are used herein to refer to one or to more than one (i.e., to at least one) of the grammatical object of the article unless the context clearly indicates otherwise. By way of example, "an element" means one element or more than one element.

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e., "one or the other but not both") when preceded by terms of exclusivity, such as "either," "one of," "only one of," or "exactly one of."

Any suitable combination(s) of any disclosed embodiments and/or any suitable portion(s) thereof are contemplated herein as appreciated by those having ordinary skill in the art in view of this disclosure.

The methods and systems of the present disclosure, as described above and shown in the drawings, provide for automatic, quick, and cheap methods of sterilization with greater probability of complete cleaning. While the apparatus and methods of the subject disclosure have been shown and described, those skilled in the art will readily appreciate that changes and/or modifications may be made thereto without departing from the scope of the invention as defined by the claims.

## Claims

1. An antiviral system comprising:
an interior space (102); and
an ultra-violet, UV, illuminator system (106) fixedly mounted within the interior space for illumination of surfaces (104) within the interior space for destruction or reduction of effect of pathogens on the surfaces.

2. The system as recited in claim 1, further comprising a plurality of seats (122) arranged within the interior space, wherein the UV illuminator system is configured to illuminate full or partial surfaces of individual seats of the plurality of seats.

3. The system as recited in any preceding claim, wherein the illuminator system includes a plurality of UV emitting lamps, or wherein the illuminator system is configured to emit at least one of UVA, UVB, and/or UVC.

4. An aircraft antiviral system comprising:
an aircraft antiviral system as claimed in any preceding claim
an aircraft defining an aircraft interior forming the interior space.

5. The antiviral system of claim 4, wherein the aircraft interior includes at least one of a cockpit (110), passenger cabin (112), lavatory (114), and/or galley (116).

6. The system as recited in claim 1, 2 or 3, wherein the interior space forms an interior of a passenger vehicle selected from the group consisting of: an aircraft, a ship, a bus, a trolleybus, a tram, a car, a train, a funicular or cable car and an elevator car., or wherein the interior space forms an interior of a stationary building selected from the group consisting of: a theater or a movie theater, an auditorium or a class room, a stadium or a sport-training room, a concert or a circus hall, and a restaurant including service and/or preparation areas.

7. The antiviral system of claim 4 or 5, wherein the plurality of UV emitting lamps are disposed along a length of an overhead bin array (132); and / or wherein the plurality of UV emitting lamps are disposed along a footpath (124) of an aisle (126); and / or wherein the plurality of UV emitting lamps are disposed along a side panel (136) of the aircraft interior space bounding an outer perimeter of a passenger row; and / or wherein the plurality of UV emitting lamps are disposed along a ceiling (130) of the aircraft interior.

8. The antiviral system of any of claims 4, 5 or 7, wherein the plurality of UV emitting lamps are positioned such that UV emitted from the plurality of UV emitting lamps is incident upon high touchpoint surfaces of the interior of the aircraft.

9. The antiviral system of claim 4 or any claim dependent thereon, wherein the illuminator system further comprises a controller (140) operatively connected to the illuminator system, the controller having machine readable instructions configured to cause the controller to perform a method, the method including:
activating the illuminator system;
sterilizing the aircraft interior with the illuminator system;
deactivating the illuminator system after a predetermined duraction of time;
boarding the aircraft;
completing aircraft flight and/or service;
de-boarding the aircraft; and
repeating the method.

10. A method for sterilizing an interior of an aircraft, comprising:
activating an illuminator system (106), the illuminator system fixedly mounted within the interior (102) of the aircraft for illumination of surfaces within the interior aircraft for destruction or reduction of effect of pathogens on the surfaces of the interior.

11. The method as recited in claim 10, further comprising boarding the aircraft with a plurality of passengers and/or crew and completing a flight and/or service of the aircraft.

12. The method as recited in claim 11, further comprising de-boarding the aircraft.

13. The method as recited in claim 12, further comprising activating the illuminator system and repeating the method.
